(19) Europäisches Patentamt

European Patent Office

Office européen des brevets

(11) **EP 0 912 641 B1**

(12) **EUROPÄISCHE PATENTSCHRIFT**

(45) Veröffentlichungstag und Bekanntmachung des Hinweises auf die Patenterteilung:
**18.06.2003 Patentblatt 2003/25**

(21) Anmeldenummer: **98910702.4**

(22) Anmeldetag: **18.02.1998**

(51) Int Cl.$^7$: **C09C 1/00**, C23C 14/06, C23C 28/00, C09D 5/36

(86) Internationale Anmeldenummer:
**PCT/EP98/00930**

(87) Internationale Veröffentlichungsnummer:
**WO 98/038254 (03.09.1998 Gazette 1998/35)**

(54) **MULTISCHICHT-INTERFERENZPIGMENT MIT TRANSPARENTER MITTELSCHICHT**

MULTILAYER INTERFERENCE PIGMENT WITH TRANSPARENT CENTRAL LAYER

PIGMENT D'INTERFERENCE A COUCHES MULTIPLES COMPORTANT UNE COUCHE CENTRALE TRANSPARENTE

(84) Benannte Vertragsstaaten:
**DE ES FI FR GB IT**

(30) Priorität: **27.02.1997 DE 19707806**

(43) Veröffentlichungstag der Anmeldung:
**06.05.1999 Patentblatt 1999/18**

(73) Patentinhaber: **MERCK PATENT GmbH**
**64271 Darmstadt (DE)**

(72) Erfinder:
• **PFAFF, Gerhard**
**D-64839 Münster (DE)**
• **BAUER, Gerd**
**D-63801 Klein-Ostheim (DE)**
• **FRIZ, Martin**
**D-64367 Mühltal (DE)**
• **KUNTZ, Matthias**
**D-64342 Seeheim (DE)**
• **SCHANK, Christina**
**D-64367 Mühltal (DE)**

(56) Entgegenhaltungen:
EP-A- 0 753 545      US-A- 5 281 480
US-A- 5 569 535

**Beschreibung**

Die Erfindung betrifft Multischicht Interferenzpigmente, bestehend aus alternierenden Schichten eines Materials mit niedriger Brechzahl und eines Metalls oder eines Materials mit hoher Brechzahl, wobei die zentrale Schicht aus einem transparenten oder semitransparenten Material mit niedriger Brechzahl gebildet wird.

Mehrschichtige Pigmente mit alternierenden Schichten aus einem Material mit hoher Brechzahl und einem Material mit niedriger Brechzahl sind bekannt. Es handelt sich hierbei vorwiegend um Metalloxide. Das Material mit hoher Brechzahl kann aber auch durch eine semitransparente Metallschicht ersetzt sein. Die Metalloxidschichten werden entweder im Maßverfahren durch Auffällen der Metalloxidhydrate aus einer Metallsalzlösung auf ein Trägermaterial oder durch Aufdampfen oder Sputtern im Vakuum hergestellt. So beschreibt US 4 434 010 ein mehrschichtiges Interferenzpigment, bestehend aus einer zentralen Schicht eines reflektierenden Materials (Aluminium) und alternierenden Schichten zweier transparenter, dielektrischer Materialien mit hoher und niedriger Brechzahl, beispielsweise Titandioxid und Siliziumdioxid beiderseits der zentralen Aluminiumschicht. In einer weiteren Ausführungsform des Pigmentes werden die auf die zentrale Aluminiumschicht folgenden Schichten durch Magnesiumfluroid und Chrom gebildet. Dieses Pigment wird für den Druck von Wertpapieren eingesetzt.

JP H7-759 (A) beschreibt ein mehrschichtiges Interferenzpigment mit metallischem Glanz. Es besteht aus einem Substrat, das mit alternierenden Schichten von Titandioxid und Siliziumdioxid beschichtet ist. Das Substrat wird aus Aluminium-, Gold- oder Silberplättchen oder Plättchen aus Glimmer und Glas, die mit Metallen beschichtet sind, gebildet. Dieses Pigment besitzt hohes Deckvermögen. Für Anwendungen, bei denen eine hohe Transparenz des pigmentierten Materials gefordert wird, beispielsweise für Agrarfolien, ist das Pigment ungeeignet. Weiterhin hat es den Nachteil, daß der typische Tiefeneffekt von Interferenzpigmenten nicht erzeugt wird, da durch die starke Reflexion des Lichtes an der den Kern bildenden Metallschicht, tiefer im Anwendungsmedium liegende Pigmentteilchen nur sehr wenig zum optischen Erscheinungsbild beitragen können. Der Interferenzeffekt bleibt deshalb auf die sich auf der Metallschicht befindenden Schichten begrenzt.

US 5,569,535 beschreibt Interferenzpigmente enthaltend reflektierende Metallschichten, die beidseitig mit einem mehrschichtigen Interferenzfilm belegt sind. Dieser Interferenzfilm setzt sich aus Paaren dielektrischer Schichten und semitransparenter Metallschichten zusammen.

Aus der EP 0 753 545 sind goniochromatische Glanzpigmente auf der Basis von mehrfach beschichteten, hochbrechenden, für sichtbares Licht teilweise durchlässigen, nichtmetallischen, plättchenförmigen Substraten bekannt. Diese Substrate sind mit einer farblosen Beschichtung mit einem Brechungsindex n ≤ 1,8 sowie einer reflektierenden, selektiv oder nichtselektiv absorbierenden, für sichtbares Licht teilweise durchlässigen Beschichtung versehen.

In US 5,281,480 wird ein Verfahren zur Herstellung optisch variabler Plättchen beschrieben, wobei ein flexibler Träger mit optisch variablen, dünnen Schichten belegt wird und die erhaltenen Pigmente anschließend vom flexiblen Träger abgetrennt werden.

Aufgabe der Erfindung ist es, ein im wesentlichen transparentes Interferenzpigment mit kräftigen Interferenzfarben und einer starken Winkelabhängigkeit der Interferenzfarben zur Verfügung zu stellen. Darüber hinaus besteht die Aufgabe der Erfindung darin, Pigmente mit speziellen, spektralen Charakteristiken im sichtbaren Bereich und im Infrarot-Bereich zur Verfügung zu stellen.

Diese Aufgabe wird gemäß der Erfindung gelöst durch ein mehrschichtiges Interferenzpigment, bestehend aus einer zentralen Schicht eines transparenten oder semitransparenten Materials mit niedriger Brechzahl und alternierenden Schichten eines Metalls oder eines Materials mit hoher Brechzahl und eines Materials mit niedriger Brechzahl beiderseits der zentralen Schicht.

Weiterhin wird diese Aufgabe gemäß der Erfindung gelöst durch ein Verfahren zur Herstellung des erfindungsgemäßen Pigmentes durch

- Aufbringen einer Ablöseschicht aus einem in Wasser oder Lösemitteln löslichen Material auf einen Träger,

- Abscheiden eines Schichtsystems aus abwechselnden Schichten eines Materials mit niedriger Brechzahl und eines Metalls oder eines Materials mit hoher Brechzahl auf die Ablöseschicht, wobei als zentrale Schicht eine Schicht aus einem transparenten oder semitransparenten Material mit niedriger Brechzahl aufgebracht wird,

- Entfernen des gebildeten Schichtsystems vom Träger durch Auflösen der Ablöseschicht, Waschen und Trocknen des erhaltenen plättchenförmigen Interferenzpigmentes,

- Wärmebehandlung des Pigments im Stickstoffstrom bei 100 bis 300 °C und

- Mahlen und Klassieren des Pigmentes.

Gegenstand der Erfindung ist außerdem die Verwendung der erfindungsgemäßen Pigmente zur Pigmentierung von Lacken, Druckfarben, Kunststoffen, Kosmetika und zur Herstellung von Folien.

Das Material mit hoher Brechzahl ist ein Metalloxid oder Mischungen von Metalloxiden ohne absorbierende Eigenschaften, wie z.B. TiO$_2$, ZrO$_2$, oder ZnO.

Das Metall ist vorzugsweise Aluminium, Chrom, Nickel, eine Chrom-Nickel-Legierung oder Silber. Chrom und Aluminium sind dabei bevorzugt, da sie leicht abzuscheiden sind. Außerdem weisen hier die Schichten eine gut zu kontrollierende Reflektivität und eine hohe Korrosionsbeständigkeit auf.

Das Material mit niedriger Brechzahl ist MgF$_2$ oder ein Metalloxid wie SiO$_2$, Al$_2$O$_3$ oder eine Mischung daraus und hat ebenfalls nicht absorbierende Eigenschaften. Gegebenenfalls kann das Material mit niedriger Brechzahl Alkali- und Erdalkalioxide als Bestandteile enthalten.

Bevorzugt werden als Material mit niedriger Brechzahl Polymere, wie z.B. Acrylate eingesetzt. Die verwendeten Monomere haben ein Molekulargewicht von 200 bis 1000 und stehen als Mono-, Di- oder Triacrylate zur Verfügung. Hinsichtlich funktioneller Gruppen sind sie als Kohlenwasserstoffe, Polyole, Polyether, Silicone oder als fluorierte teflonartige Monomere verfügbar. Diese Monomere können durch Elektronenstrahlen oder UV-Strahlen polymerisiert werden. Die erhaltenen Schichten besitzen eine Temperaturstabilität bis zu 250 °C. Die Brechzahlen der Acrylatschichten liegen im Bereich von 1,35 bis 1,60. Weitere Angaben sind bei David G. Shaw und Marc G. Langlois zu finden: Use of a new high speed acrylate deposition process to make novel multilayer structures, MRS-Tagung in San Francisco 1995; A new high speed process for vapor depositing fluoro and silicone acrylates for release coating applications, Tagung der Society of Vacuum Coater in Chicago, Illinois, 1995.

Die Differenz der Brechzahlen zwischen einer Schicht mit hoher Brechzahl und einer Schicht mit niedriger Brechzahl sollte mindestens 0,1 betragen.

Die Schichtdicke der Schichten mit niedriger und hoher Brechzahl wird auf Werte zwischen 20 nm und 700 nm, bevorzugt zwischen 60 nm und 500 nm eingestellt. Die Schichtdicke der Metallschichten wird auf 5 bis 20 nm eingestellt, um Semitransparenz zu erhalten.

Die mit einem Vielfachschichtsystem maximal erreichbare Reflexion hängt von der Schichtzahl und den Brechzahlen der Schichten ab:

$$R = \left[ \frac{1-(n_H/n_L)^{2p}\, n_H^2}{1+(n_H/n_L)^{2p}\, n_H^2} \right]^2$$

Dabei ist $n_H$ die Brechzahl der hochbrechenden Schicht, $n_L$ die Brechzahl der niedrigbrechenden Schicht und P ist die Schichtzahl. Diese Gleichung gilt für eine Schichtzahl von 2p + 1.

Die Schichtdicke für maximale Reflexion ist jeweils d = λ/4n oder ein Vielfaches davon mit der Lichtwellenlänge. Die Dicke und Anzahl der Schichten richtet sich nach dem gewünschten Effekt hinsichtlich Interferenzfarbe und Winkelabhängigkeit der Interferenzfarbe. λ bewegt sich im Bereich zwischen 400 nm (violettes Licht) bis etwa 750 nm (roter Bereich). Um entsprechende Farben zu bekommen, muß die Schichtdicke, abhängig von der Brechzahl des optisch dünneren Mediums eingestellt werden. Darüber hinaus kann man die erfindungsgemäßen Pigmente auch dazu einsetzen, in den angrenzenden Wellenbereichen - (UV - Infrarot) - entsprechende selektiv reflektierende Pigmente herzustellen.

In der Feinoptik, z.B. bei der Herstellung von Spiegelschichten, Strahlteilern oder Filtern wird mit Schichtzahlen bis zu 100 oder mehr gearbeitet. Für die Herstellung von Pigmenten sind derartig hohe Schichtzahlen nicht notwendig. Die Anzahl der Schichten liegt normalerweise unter 10.

Die einzelnen Schichten werden nach bekannten Verfahren durch Sputtern von Metallen, beispielsweise von Aluminium oder Chrom oder von Legierungen, wie zum Beispiel Chrom-Nickel-Legierungen sowie von Metalloxiden, beispielsweise von Titanoxid, Siliciumoxid oder Indium-Zinn-Oxid oder durch thermisches Verdampfen von Metallen, Metalloxiden oder Acrylaten hergestellt.

Bevorzugt wird für die Herstellung der erfindungsgemäßen Pigmente eine Vakuumbandbeschichtung, wie sie in US 5 440 446 für die Herstellung von Hochspannungskondensatoren und in EP 0 733 919 für die Herstellung von Interferenzfarbfiltern beschrieben wird.

Als Träger für das Interferenzschichtsystem wird ein flexibles Band aus Polyethylenterephthalat (PET), anderen Polyestern, Polyacrylaten, Polyethylen (PE) oder Polypropylen (PP) verwendet.

Die Ablöseschicht, die auf den Träger aufgebracht wird, um das Interferenzschichtsystem nach erfolgter Abscheidung vom flexiblen Band ablösen zu können, besteht aus einem in Wasser oder Lösemitteln löslichen Material, wie z.B. Polyethylenglykol, Wachs oder Silikon. Als Lösungsmittel werden Wasser oder Aceton verwendet.

Im folgenden soll das Aufbringen der Interferenzschichten durch Aufdampfen näher beschrieben werden:

Bei den Aufdampfverfahren werden die zu verdampfenden Stoffe im Vakuum erhitzt und verdampft. Die Dämpfe kondensieren auf den kalten Substratflächen zu den gewünschten dünnen Schichten. Die Verdampfung geschieht entweder in Behältern aus Metall (Schiffchen aus Wolfram, Molybdän oder Tantalblech), die durch Stromdurchgang direkt beheizt werden, oder durch Beschuß mit Elektronenstrahlen.

Für die Herstellung des Interferenzschichtsystems kann eine herkömmliche Bandbedampfungsanlage eingesetzt werden. Die Aufdampfanlage besteht aus den üblichen Komponenten, wie Vakuumkessel, Vakuumpumpsystem, Druckmeß- und Steuereinheiten, Verdampfereinrichtungen, wie Widerstandsverdampfer (Schiffchen) oder Elektronenstrahlverdampfer, Schichtdickenmeß- und Kontrollsystem, Vorrichtung zur Einstellung bestimmter Druckverhältnisse sowie ein Gaseinlaß- und Regelsystem für Sauerstoff.

Die Hochvakuumaufdampftechnik ist ausführlich beschrieben in Vakuum-Beschichtung, Bände 1-5; Herausgeber Frey, Kienel u. Löbl, VDI-Verlag 1995.

Das Aufbringen der Schichten durch Sputter-Verfahren erfolgt auf folgende Weise:

Beim Sputterverfahren oder bei der Kathodenzerstäubung wird zwischen dem Träger und dem Beschichtungsmaterial, das in Form von Platten (Target) vorliegt, eine Gasentladung (Plasma) gezündet. Das Beschichtungsmaterial wird durch energiereiche Ionen aus dem Plasma, z.B. Argonionen, beschossen und dadurch abgetragen bzw. zerstäubt. Die Atome oder Moleküle des zerstäubten Beschichtungsmaterials werden auf dem Träger niedergeschlagen und bilden die gewünschte dünne Schicht.

Für Sputterverfahren eignen sich besonders Metalle oder Legierungen. Diese können mit vergleichsweise hohen Geschwindigkeiten, insbesondere im sogenannten DC-Magnetron-Verfahren, zerstäubt werden. Verbindungen wie Oxide oder Suboxide oder Mischungen aus Oxiden können durch Einsatz des Hochfrequenz-Sputtern ebenfalls zerstäubt werden. Die chemische Zusammensetzung der Schichten wird durch die Zusammensetzung des Beschichtungsmaterials (Target) bestimmt. Sie kann aber auch durch Zusätze zum Gas, das das Plasma bildet, beeinflußt werden. Insbesondere werden Oxid- oder Nitritschichten durch Zusatz von Sauerstoff oder Stickstoff im Gasraum hergestellt.

Die Struktur der Schichten kann durch geeignete Maßnahmen, wie Beschuß der aufwachsenden Schicht durch Ionen aus dem Plasma, beeinflußt werden.

Das Sputterverfahren ist ebenfalls beschrieben in Vakuum-Beschichtung, Bände 1-5; Herausgeber Frey, Kienel und Löbl, VDI-Verlag 1995.

Das Prinzip des Aufbringens der Schichten ist in US 5 440 446 und EP 0 733 919 beschrieben und erfolgt auf folgende Weise:

Die gesamte Beschichtungsanlage befindet sich innerhalb einer konventionellen Vakuumkammer 1. Auf einer Abspulrolle 2 ist ein Polyesterband aufgewickelt, auf das bereits auf einer Seite eine Metallschicht aufgebracht ist. Vor der Metallisierung wurde das Polyesterband mit einer Ablöseschicht versehen. Das Polyesterband 3 wird über eine rotierende Trommel 4 geführt und auf die Aufnahmerolle 5 aufgewickelt. Rolle 6 und Rolle 7 dienen als Spann- und Führungsrollen.

Das Band passiert die Metallisierungsstation 8, wo eine semitransparente Metallschicht durch Vakumverdampfung oder Sputtern abgeschieden wird. An-schließend passiert das Band den Schnellverdampfer 9. Im Verdampfer befindet sich ein gasförmiges Acrylatmonomer, das als dünne Schicht auf die auf dem Trägerband befindliche Metallschicht abgeschieden wird. Das Band durchläuft anschließend eine Bestrahlungsstation 10, wo es mit Elektronen oder ultraviolettem Licht bestrahlt wird. Durch die Bestrahlung wird die Polymerisation des Acrylatmonomers ausgelöst. Dann passiert das Band die zweite Metallisierungsstation 11. Bei Station 12 wird eine weitere Acrylatmonomerschicht aufgebracht, die in Bestrahlungsstation 13 polymerisiert wird in Analogie zu den in den Stationen 9 und 10 ablaufenden Schritten. Danach wird auf das Band, das mit zwei semitransparenten Metallschichten und zwei zwischen ihnen angeordneten Acrylatschichten beschichtet ist, nach Passieren von Spannrolle 7 aufgewickelt.

Danach durchläuft das Band ein zweites Mal die Vakuumanlage, wo in gleicher Weise wie beim ersten Durchlauf die Metallschichten und die Acrylatschicht abgeschieden werden, allerdings sind diesmal die Stationen 12 und 13 außer Betrieb.

Für ein Schichtsystem aus 7 Schichten, bestehend aus einer zentralen Absorptionsschicht und zwei Metallschichten und einer Acrylatschicht beiderseits der zentralen Schicht, sind 2 Durchläufe durch die Vakuumbeschichtungsanlage erforderlich.

Nach der Beschichtung erfolgt das Ablösen der Mehrfachbeschichtung durch Auflösen der Ablöseschicht in einem Wasserbad, ggf. bei höherer Temperatur, oder in einem Lösemittel, ggf. bei höherer Temperatur, durch Abbürsten, Abschaben oder vorzugsweise durch Abspülen.

Bei Verwendung von Acrylaten als niedrigbrechendes Material muß das Mahlen des Pigmentes bei tieferen Temperaturen im Bereich von 90 bis 273 K erfolgen.

Das nachfolgende Beispiel soll die Erfindung erläutern.

**Beispiel 1**

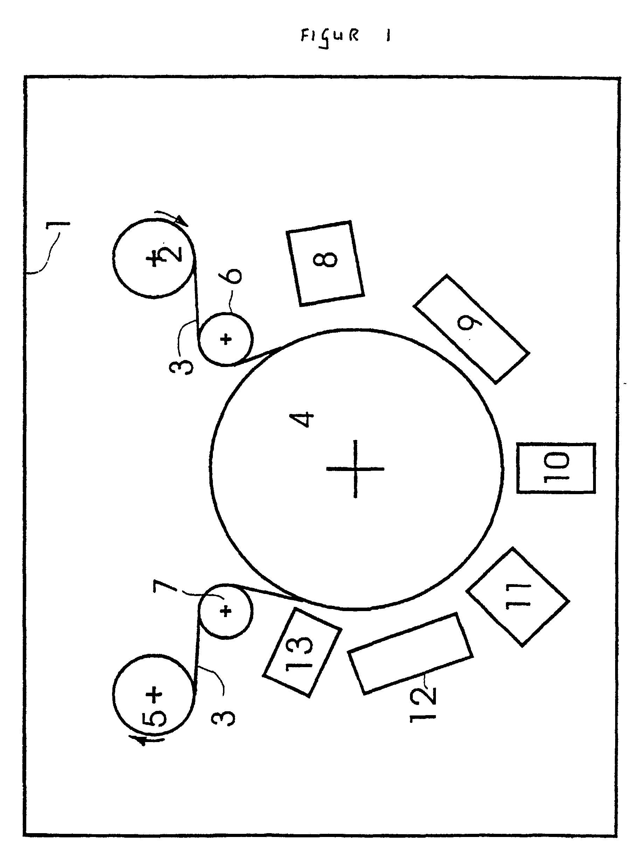

Ein Interferenzpigment, bestehend aus 7 Schichten, wird durch abwechselndes Aufdampfen von Chrom bzw. Aluminium und Acrylat auf ein Polyesterband in einer Vakuumbedampfungsanlage gemäß Figur 1 hergestellt. Das Polyesterband ist mit einer Ablöseschicht aus Stearin beschichtet. Nach dem Aufdampfen von zwei Chrom- und zwei Acrylatschichten, wird das Band ein zweites Mal durch die Vakuumanlage geschickt, wo anders als beim ersten Durchlauf zwei Chromschichten und nur eine Acrylatschicht abgeschieden werden.

| Schichtaufbau des Pigmentes | | |
|---|---|---|
| *Schicht- Nr.* | *Material* | *Schichtdicke nm* |
| 1 | Chrom | 17 |

(fortgesetzt)

| Schichtaufbau des Pigmentes | | |
|---|---|---|
| *Schicht- Nr.* | *Material* | *Schichtdicke nm* |
| 2 | Acrylat | 325 |
| 3 | Chrom | 17 |
| 4 | Acrylat | 325 |
| 5 | Chrom | 17 |
| 6 | Acrylat | 325 |
| 7 | Chrom | 17 |

Das Schichtsystem wird mit Aceton vom Trägerband abgelöst, mit Aceton gewaschen und getrocknet. Anschließend wird das erhaltene Pigment im Stickstoffstrom für 90 min auf 300 °C erwärmt und nachfolgend in einer Mörsermühle der Firma Netsch 30 min, gemischt mit Kohlendioxid-Trockeneis, bei -5 bis -10 °C auf eine mittlere Teilchengröße von 40 μm zerkleinert.

**Beispiel 2**

Ein Interferenzpigment, bestehend aus 7 Schichten, wird durch abwechselndes Aufdampfen von Chrom bzw. Silber und Magnesiumfluorid auf eine Folie aus Polyethylenterephthalat hergestellt. Die Folie ist mit einer Ablöseschicht aus Stearin beschichtet. Das Aufdampfen erfolgt in einer Hochvakuum-Aufdampfanlage A 700 Q der Firma Leybold AG.

| Schichtaufbau des Pigmentes | | |
|---|---|---|
| *Schicht- Nr.* | *Material* | *Schichtdicke nm* |
| 1 | Cr | 5 |
| 2 | $MgF_2$ | 453 |
| 3 | Ag | 10 |
| 4 | $MgF_2$ | 90 |
| 5 | Ag | 10 |
| 6 | $MgF_2$ | 453 |
| 7 | Cr | 5 |

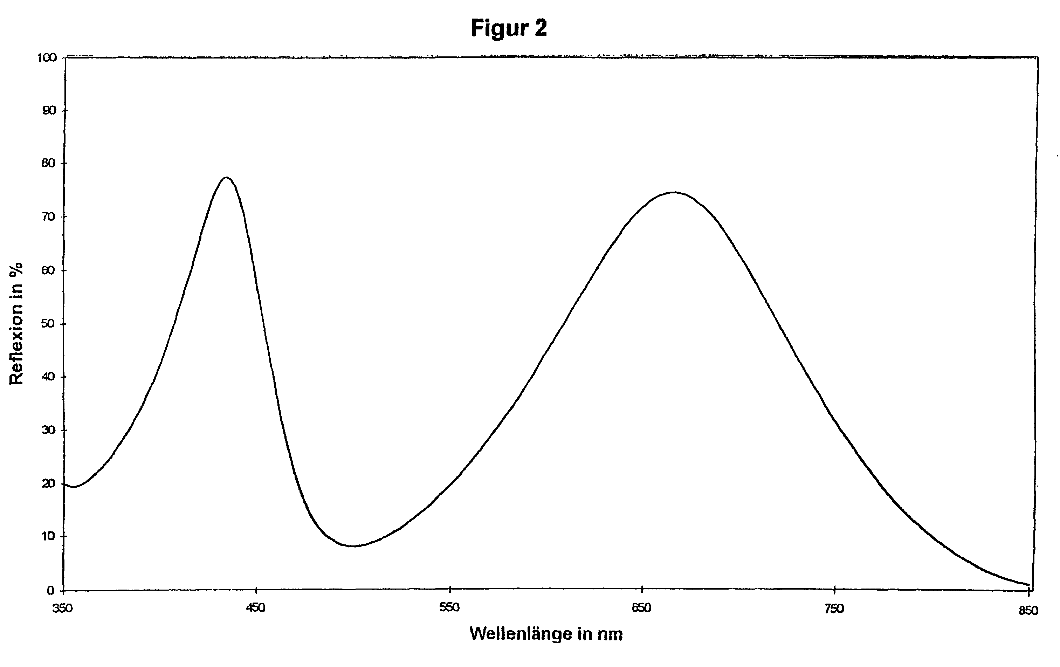

Das Schichtsystem wird mit Aceton von der Folie abgelöst, mit Aceton gewaschen, getrocknet und in einer Mörsermühle der Firma Netsch 30 min gemahlen. Man erhält ein Pigment mit einer mittleren Teilchengröße von 40 μm.
Das Reflexionsspektrum ist in Figur 2 dargestellt.

**Patentansprüche**

1. Multischicht-Interferenzpigment, bestehend aus einer zentralen Schicht eines transparenten oder semitransparenten Materials mit niedriger Brechzahl und alternierenden Schichten eines Metalls oder eines Metalloxides ohne absorbierende Eigenschaften mit hoher Brechzahl und eines Materials ohne absorbierende Eigenschaften mit niedriger Brechzahl.

2. Interferenzpigment nach Anspruch 1, **dadurch gekennzeichnet, daß** das Material mit niedriger Brechzahl $MgF_2$, ein Metalloxid oder ein Polymer ist.

3. Interferenzpigment nach Anspruch 2, **dadurch gekennzeichnet, daß** das Polymer ein Acrylat ist.

4. Interferenzpigment nach Anspruch 2, **dadurch gekennzeichnet, daß** das Metalloxid $SiO_2$, $Al_2O_3$ oder eine Mischung daraus ist.

5. Interferenzpigment nach Anspruch 1, **dadurch gekennzeichnet, daß** das Metall Aluminium, Chrom, Nickel, eine Ni-Cr-Legierung oder Silber ist.

6. Interferenzpigment nach Anspruch 1, **dadurch gekennzeichnet, daß** das Metalloxid mit hoher Brechzahl $TiO_2$, $ZrO_2$, ZnO oder ein Gemisch aus diesen Oxiden ist.

7. Verfahren zur Herstellung des Interferenzpigmentes nach den Ansprüchen 1 bis 6, **dadurch gekennzeichnet, daß**

   - eine Ablöseschicht aus einem in Wasser oder Lösemitteln löslichen Material auf einen Träger aufgebracht wird,

   - ein Schichtsystem aus abwechselnden Schichten eines Materials ohne absorbierende Eigenschaften mit niedriger Brechzahl und eines Metalls oder eines Metalloxides ohne absorbierende Eigenschaften mit hoher Brechzahl auf die Ablöseschicht abgeschieden wird, wobei als zentrale Schicht eine Schicht aus einem transparenten oder semitransparenten Material mit niedriger Brechzahl aufgebracht wird,

   - das gebildete Schichtsystem vom Träger durch Auflösen der Ablöseschicht entfernt wird und das erhaltene plättchenförmige Interferenzpigment gewaschen und getrocknet wird,

   - das Pigment im Stickstoffstrom einer Wärmebehandlung bei 100 bis 300 °C unterzogen wird und

   - gemahlen und klassiert wird.

8. Verfahren nach Anspruch 7, **dadurch gekennzeichnet, daß** das Material ohne absorbierende Eigenschaften mit niedriger Brechzahl $MgF_2$, ein Metalloxid oder ein Polymer ist.

**9.** Verfahren nach Anspruch 8, **dadurch gekennzeichnet, daß** das Polymer ein Acrylat ist.

**10.** Verfahren nach Anspruch 8, **dadurch gekennzeichnet, daß** das Metalloxid SiO$_2$, Al$_2$O$_3$ oder eine Mischung daraus ist.

**11.** Verfahren nach Anspruch 7, **dadurch gekennzeichnet, daß** das Metall Aluminium, Chrom, Nikkel, eine Ni-Cr-Legierung oder Silber ist.

**12.** Verfahren nach Anspruch 7, **dadurch gekennzeichnet, daß** das Metalloxid ohne absorbierende Eigenschaften mit hoher Brechzahl TiO$_2$, ZrO$_2$, ZnO oder ein Gemisch aus diesen Oxiden ist.

**13.** Verwendung der Pigmente nach den Ansprüchen 1 bis 6 zur Pigmentierung von Lacken, Druckfarben, Kunststoffen und Kosmetika.

**14.** Verwendung nach Anspruch 13, **dadurch gekennzeichnet, daß** die Pigmente als Mischungen mit konventionellen Pigmenten und mit anderen Effektpigmenten eingesetzt werden.

**15.** Lacke, Druckfarben, Kunststoffe und Kosmetika, welche mit einem Pigment nach den Ansprüchen 1 bis 6 pigmentiert sind.

**Claims**

**1.** Multilayer interference pigment consisting of a central layer of a transparent or semitransparent material of low refractive index and alternating layers of a metal or of a metal oxide without absorbing properties and of high refractive index and of a material without absorbing properties and of low refractive index.

**2.** Interference pigment according to Claim 1, **characterized in that** the material of low refractive index is MgF$_2$, a metal oxide or a polymer.

**3.** Interference pigment according to Claim 2, **characterized in that** the polymer is an acrylate.

**4.** Interference pigment according to Claim 2, **characterized in that** the metal oxide is SiO$_2$, Al$_2$O$_3$ or a mixture thereof.

**5.** Interference pigment according to Claim 1, **characterized in that** the metal is aluminium, chromium, nickel, a Ni-Cr alloy or silver.

**6.** Interference pigment according to Claim 1, **characterized in that** the metal oxide of high refractive index is TiO$_2$, ZrO$_2$, ZnO or a mixture of these oxides.

**7.** Process for preparing the interference pigment according to Claims 1 to 6, **characterized in that**

- a release layer comprising a water- or solvent-soluble material is applied to a substrate,

- a layer system comprising alternating layers of a material without absorbing properties and of low refractive index and of a metal or of a metal oxide without absorbing properties and of high refractive index is deposited onto the release layer, the central layer applied being a layer of a transparent or semitransparent material of low refractive index,

- the layer system formed is removed from the substrate by dissolving the release layer, and the resulting platelet-shaped interference pigment is washed and dried,

- the pigment is subjected to heat treatment at from 100 to 300°C, in a stream of nitrogen, and

- the treated pigment is milled and classified.

**8.** Process according to Claim 7, **characterized in that** the material without absorbing properties and of low refractive index is MgF$_2$, a metal oxide or a polymer.

**9.** Process according to Claim 8, **characterized in that** the polymer is an acrylate.

**10.** Process according to Claim 8, **characterized in that** the metal oxide is SiO$_2$, Al$_2$O$_3$ or a mixture thereof.

**11.** Process according to Claim 7, **characterized in that** the metal is aluminium, chromium, nickel, a Ni-Cr alloy or silver.

**12.** Process according to Claim 7, **characterized in that** the metal oxide without absorbing properties and of high refractive index is TiO$_2$, ZrO$_2$, ZnO or a mixture of these oxides.

**13.** Use of the pigments according to Claims 1 to 6 for pigmenting paints, printing inks, plastics and cosmetics.

**14.** Use according to Claim 13, **characterized in that** the pigments are employed as mixtures with conventional pigments and with other special-effect pigments.

**15.** Paints, printing inks, plastics and cosmetics which have been pigmented with a pigment according to Claims 1 to 6.

**Revendications**

1. Pigment d'interférence multicouche, constitué d'une couche centrale d'un matériau transparent ou semk-transparent ayant un indice de réfraction faible et de couches en alternance d'un métal ou d'un oxyde métallique sans propriétés absorbantes ayant un indice de réfraction élevé et d'un matériau sans propriétés absorbantes ayant un indice de réfraction faible.

2. Pigment d'interférence selon la revendication 1, **caractérisé en ce que** le matériau ayant un indice de réfraction faible est MgF$_2$, un oxyde métallique ou un polymère.

3. Pigment d'interférence selon la revendication 2, **caractérisé en ce que** le polymère est un acrylate.

4. Pigment d'interférence selon la revendication 2, **caractérisé en ce que** l'oxyde métallique est SiO$_2$, Al$_2$O$_3$ ou un mélange de ceux-ci.

5. Pigment d'interférence selon la revendication 1, **caractérisé en ce que** le métal est l'aluminium, le chrome, le nickel, un alliage Ni-Cr ou l'argent.

6. Pigment d'interférence selon la revendication 1, **caractérisé en ce que** l'oxyde métallique ayant un indice de réfraction élevé est TiO$_2$, ZrO$_2$, ZnO ou un mélange constitué de ces oxydes.

7. Procédé pour la préparation du pigment d'interférence selon les revendications 1 à 6, **caractérisé**

   - **en ce que** l'on applique sur un substrat une couche de séparation constituée d'un matériau soluble dans l'eau ou les solvants,
   - **en ce que** l'on dépose sur la couche de séparation un système de couches constitué de couches en alternance d'un matériau sans propriétés absorbantes ayant un indice de réfraction faible et d'un métal ou d'un oxyde métallique sans propriétés absorbantes ayant un indice de réfraction élevé, une couche constituée d'un matériau transparent ou semitransparent ayant un indice de réfraction faible étant appliquée comme couche centrale,
   - **en ce que** l'on sépare du substrat le système de couches formé, par dissolution de la couche de séparation et en ce que l'on lave le pigment d'interférence sous forme lamellaire obtenu et en ce que l'on le sèche,
   - **en ce que** l'on soumet le pigment à un courant d'azote à un traitement thermique à une température comprise entre 100 et 300°C et
   - **en ce que** l'on broie et en ce que l'on calibre.

8. Procédé selon la revendication 7, **caractérisé en ce que** le matériau sans propriétés absorbantes ayant un indice de réfraction faible est MgF$_2$, un oxyde métallique ou un polymère.

9. Procédé selon la revendication 8, **caractérisé en ce que** le polymère est un acrylate.

10. Procédé selon la revendication 8, **caractérisé en ce que** l'oxyde métallique est SiO$_2$, Al$_2$O$_3$ ou un mélange de ceux-ci.

11. Procédé selon la revendication 7, **caractérisé en ce que** le métal est l'aluminium, le chrome, le nickel, un alliage Ni-Cr ou l'argent.

12. Procédé selon la revendication 7, **caractérisé en ce que** l'oxyde métallique sans propriétés absorbantes ayant un indice de réfraction élevé est TiO$_2$, ZrO$_2$, ZnO ou un mélange constitué de ces oxydes.

13. Utilisation des pigments selon les revendications 1 à 6 pour la pigmentation des vernis, encres d'imprimerie, matières plastiques et produits cosmétiques.

14. Utilisation selon la revendication 13, **caractérisée en ce que** les pigments sont utilisés sous forme de mélanges avec des pigments classiques et avec d'autres pigments à effets spéciaux.

15. Vernis, encres d'imprimerie, matières plastiques et produits cosmétiques, pigmentés avec un pigment selon les revendications 1 à 6.

FIGUR 1

**Figur 2**

9